# EUROPEAN PATENT APPLICATION

(11) **EP 4 328 234 A1**
(43) Date of publication of application: **28.02.2024**
(21) Application number: 22791023.9
(22) Date of filing: 19.04.2022
(51) Int. Cl.: C07K 5/037, A61K 38/06, A61P 1/16

(54) **COMPOUND FOR ALCOHOLIC LIVER INJURY, PREPARATION METHOD, COMPOSITION, FOOD AND USE**

(30) Priority: 20.04.2021 CN 202110425975; 26.04.2021 CN 202110456181; 11.04.2022 CN 202210374114
(71) Applicant: Chengdu Innoheporem Biotech Co., Ltd., Chengdu, Sichuan 610000 (CN)
(72) Inventor: KANG, Yujian James, Chengdu, Sichuan 610000 (CN)
(74) Representative: Eisenführ Speiser
(86) International application number: PCT/CN2022/087637
(87) International publication number: WO 2022/222916

(57) **Abstract**

Provided are a compound for alcoholic liver injury, a preparation method, a composition, a food and the use. The compound has the following formula (I), wherein M is a divalent metal cation selected from Cu, Fe and Zn; A is selected from glutathione and a derivative thereof, and the derivative of glutathione is selected from: at least one of amino or carboxyl in glutathione substituted with an amino protecting group and/or a carboxyl protecting group; B is a monovalent or divalent balance anion; and n is 1 or 2.

[M(A)₂]·nB (I)

## Description

### CROSS REFERENCE TO RELATED APPLICATIONS

This application claims the priority of Chinese Patent Application No. 202110425975.9, filed with the China National Intellectual Property Administration on April 20, 2021, and titled with "COMPOUND FOR ALCOHOLIC LIVER INJURY, PREPARATION METHOD, COMPOSITION, FOOD AND USE", Chinese Patent Application No. 202110456181.9, filed with the China National Intellectual Property Administration on April 26, 2021, and titled with "COMPOUND FOR ALCOHOLIC LIVER INJURY, PREPARATION METHOD, COMPOSITION, FOOD AND USE" and Chinese Patent Application No. 202210374114.7, filed with the China National Intellectual Property Administration on April 11, 2022, and titled with "COMPOUND FOR ALCOHOLIC LIVER INJURY, PREPARATION METHOD, COMPOSITION, FOOD AND USE", which are hereby incorporated by reference in their entirety.

### FIELD

The present invention relates to the field of medicine, and in particular to a drug and a food for alcoholic liver injury.

### BACKGROUND

Liver is the largest digestive gland and the largest detoxification organ in the human body. China is a country with a large number of people drink alcohol and have liver disease.

Alcohol is widely considered to have toxic effects on the liver. Alcoholic liver injury refers to liver diseases caused by long-term heavy drinking of alcohol or alcoholic beverages, including alcoholic fatty liver, alcoholic hepatitis, alcoholic hepatic fibrosis, mild alcoholic liver disease, alcoholic cirrhosis, etc. Alcoholic liver injury often begins with alcoholic fatty liver (AFL), which is characterized by hepatic steatosis (triglycerides accumulate in liver cells). Some people develop liver inflammation, hepatocyte damage, and hepatocyte ballooning, which is histologically defined as alcoholic steatohepatitis (ASH). ASH progresses slowly, and with ongoing chronic liver injury and inflammation eventually leading to progressive fibrosis and cirrhosis, it may ultimately lead to the development of hepatocellular carcinoma (HCC).

In the past 20 years, compared with the decline in new cases of viral hepatitis brought about by the popularization of hepatitis B vaccines and the application of antiviral drugs, the improvement of the quality of life of Chinese people has directly led to alcohol abuse, which ultimately led to the rapid increase in the incidence rate of alcoholic liver disease (ALD) in China. According to a survey by Gao B et al. in 2011, about 90% of heavy drinkers (who consume more than 60 grams of alcohol per day) will develop fatty liver, 10%-35% will develop alcoholic hepatitis, and 5%-15% will develop cirrhosis. A survey by the US NIAAA/NIH shows that 48% of patients who died from liver cirrhosis in 2007 alone were alcohol-related. The number of alcohol-related deaths in China has increased over the past two decades, accounting for 30.2% of the total annual deaths. In 2013, 590,000 people died as a result of drinking, more than half of whom were men.

There are a large number of liver-protecting health products/foods that are used to alleviate the toxicity of alcohol to the liver during or after drinking. Such products typically include antioxidants, trace minerals, traditional liver-protecting medications/foods, and the like.

There is still a lack of novel and conveniently administered (e.g., orally) effective drugs for the prevention, alleviation, or treatment of established alcoholic liver injury. For dietary products that may cause alcoholic liver injury, there is also a need for related products that are more effective in preventing or alleviating alcoholic liver injury.

### SUMMARY

In view of this, the main purpose of the present invention is to provide a compound that can effectively prevent, alleviate or treat alcoholic liver injury. The compound is easy to prepare, has oral activity, and has significant effects on alcoholic liver injury. A further purpose of the present invention is to provide a method for preparing the compound, a pharmaceutical composition or food comprising the compound, and use of the compound, pharmaceutical composition and food.

Therefore, a first aspect of the present application provides a compound represented by general formula I:

[M(A)₂]·nB (I)

wherein, M is a divalent metal cation selected from the group consisting of Cu, Fe and Zn;
A is selected from the group consisting of glutathione and a glutathione derivative, wherein the glutathione derivative is selected from the group consisting of glutathione in which at least one of the amino group or carboxyl group is substituted by an amino protecting group and/or a carboxyl protecting group;
B is a monovalent or divalent counter anion; and
n is 1 or 2.

According to an embodiment, B may be a sulfate or halide ion. The halide ion may be a chloride ion or a bromide ion.

According to an embodiment, the glutathione and glutathione derivative are formed from an L-type amino acid.

In particular, the compound can be selected from the group consisting of [Zn²⁺(L-GSH)₂]·SO₄²-, [Zn²¹(L-GSH)₂]·2Cl-, [Cu²⁺(L-GSH)₂]·SO₄²⁻, [Cu²⁺(L-GSH)₂]·2Cl⁻, [Fe²⁺(L-GSH)₂]·SO₄²⁻ and [Fe²⁺(L-GSH)₂]·2Cl⁻.

A second aspect of the present application provides a method for preparing the above compound, comprising mixing A and an inorganic salt MBₙ of M in an aqueous solvent at a molar ratio of A to MBₙ of 2: (1-1.2) for reaction, wherein A, M, B and n are defined as above.

According to a specific embodiment, after the reaction is completed, distillation under reduced pressure is performed to obtain a concentrated solution, which is further purified to obtain the compound.

According to a specific embodiment, the inorganic salt MBₙ of the divalent metal ion M may be a hydrohalide (such as a hydrochloride) or a sulfate salt of the metal ion M.

According to a specific embodiment, the purification may comprise dissolving the obtained concentrated solution with a good solvent (such as water), then adding a poor solvent (such as alcohol) to crystallize and precipitate the compound, then filtering the solution, and washing and drying the precipitate to obtain the compound represented by formula (I).

A third aspect of the present application provides a pharmaceutical composition comprising a therapeutically effective amount for treating alcoholic liver injury of the above compound.

A fourth aspect of the present invention provides a food comprising at least one of the above compounds.

The food is a wine.

According to a specific embodiment, the wine comprises 0.6~0.8 g/L, preferably 0.7~0.8 g/L of the above compound.

A fifth aspect of the present invention provides use of the above compound or the above pharmaceutical composition in the manufacture of a medicament for preventing, alleviating or treating alcoholic liver injury.

It can be seen from the examples that will be described in detail below that the glutathione-based metal chelate of the present invention can effectively reduce alcoholic liver injury.

### BRIEF DESCRIPTION OF DRAWINGS

FIG. 1 shows the changes in the reactants and products in the reaction system during the reaction process of preparing L-glutathione zinc chelate in Example 1 monitored by HPLC;
FIG. 2 shows the infrared spectra of L-GSH (A) and the product of Example 1 (B);
FIG. 3 shows the mass spectra of L-GSH (A) and the product of Example 1 (B);
FIG. 4 shows the animal grouping and processing flow chart in Example 4;
FIG. 5 shows the microscopic photos of oil red O-stained liver tissue sections of mice fed normally for three months, and mice fed with alcohol for three months and six months in Example 3;
FIG. 6 shows the statistical analysis of the percentage of fat deposition in the total liver area calculated based on the oil red O-stained liver tissue sections of mice fed normally for three months, and mice fed with alcohol for three months and six months in Example 3;
FIG. 7 shows the microscopic photos of HE-stained liver tissue sections of mice fed normally for three months, and mice fed with alcohol for three months and six months in Example 3;
FIG. 8 shows the survival statusof mice fed normally for six months, mice fed with alcohol for six months without intervention, and mice fed with alcohol for six months and followed by three months of intervention in Example 4;
FIG. 9 shows the microscopic photos of oil red O-stained liver tissue sections of mice fed normally for six months, mice fed with alcohol for six months without intervention, and mice fed with alcohol for six months and followed by three months of intervention in Example 4;
FIG. 10 shows the statistical analysis of the percentage of fat deposition in the total liver area calculated based on the oil red O-stained liver tissue sections of mice fed normally for six months, mice fed with alcohol for six months without intervention, and mice fed with alcohol for six months and followed by three months of intervention in Example 4;
FIG. 11 shows microscopic photos of HE-stained liver tissue sections of mice in each group in Example 4;
FIG. 12 shows the measurement results of zinc content in the livers of mice fed normally for six months, mice fed with alcohol for six months without intervention, and mice fed with alcohol for six months and followed by three months of intervention in Example 4;
FIG. 13 shows the animal grouping and processing flow chart in Example 6;

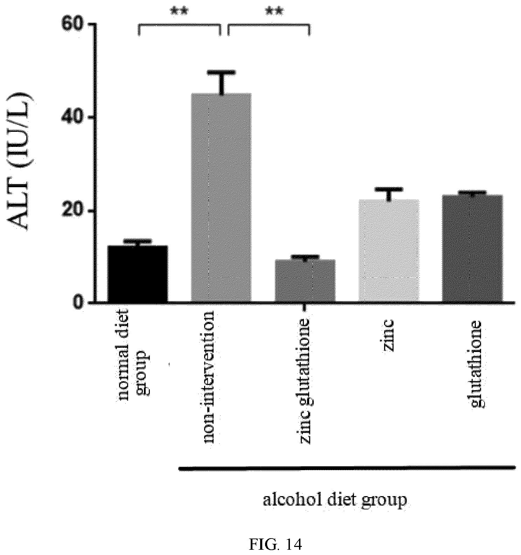

FIG. 14 shows the measurement results of alanine aminotransferase (ALT) in the serum of mice fed normally for seven months (normal diet group), mice fed with alcohol for six months and then fed normally for one month (no intervention group), mice fed with alcohol for six months and then fed with zinc glutathione feed for one month (zinc glutathione intervention group), mice fed with alcohol for six months and then fed with zinc feed for one month (zinc intervention group), and mice fed with alcohol for six months and fed with glutathione feeding for one month (glutathione intervention group) in Example 6;

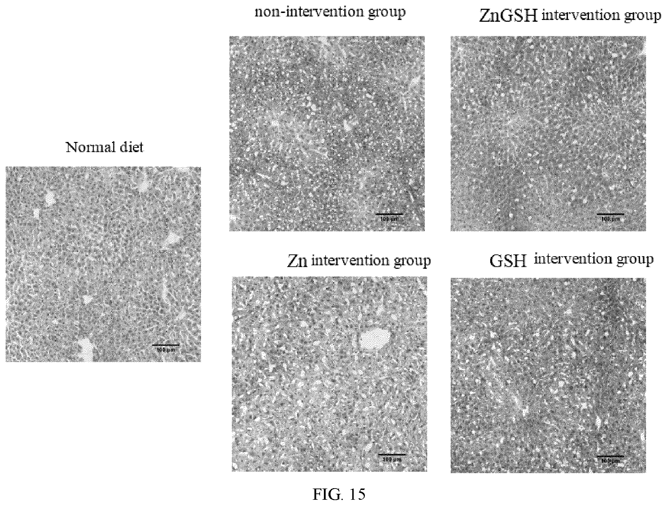

FIG. 15 shows the microscopic photos of oil red O-stained liver tissue sections of mice fed normally for seven months (normal diet group), mice fed with alcohol for six months and then fed normally for one month (no intervention group), mice fed with alcohol for six months and then fed with zinc glutathione feed for one month (zinc glutathione intervention group), mice fed with alcohol for six months and then fed with zinc feed for one month (zinc intervention group), and mice fed with alcohol for six months and then fed with glutathione feeding for one month (glutathione intervention group) in Example 6;

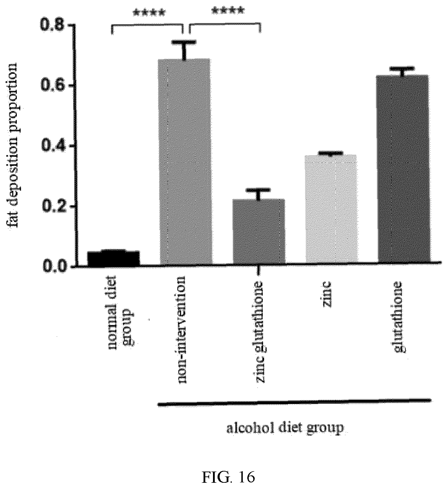

FIG. 16 shows the statistical analysis of the percentage of fat deposition in the total liver area calculated based on the oil red O-stained liver tissue sections of mice fed normally for seven months (normal diet group), mice fed with alcohol for six months and fed normally for one month (no intervention group), mice fed with alcohol for six months and fed with zinc glutathione feed for one month (zinc glutathione intervention group), mice fed with alcohol for six months and fed with zinc feed for one month (zinc intervention group), and mice fed with alcohol for six months and fed with glutathione feeding for one month (glutathione intervention group) in Example 6.

### DETAILED DESCRIPTION

The technical solutions in the embodiments of the present invention will be clearly and completely described below with reference to the embodiments of the present invention and the drawings. Apparently, the described embodiments are only some of the embodiments of the present invention, but not all of them. Based on the embodiments of the present invention, all other embodiments obtained by those of ordinary skill in the art without making inventive efforts fall within the scope of protection of the present invention.

Throughout this specification, unless otherwise specifically stated, the terms used herein are to be understood as having the meaning commonly used in the art. Therefore, unless otherwise defined, all technical and scientific terms used herein have the same meaning as commonly understood by those of ordinary skill in the art to which the present invention belongs. If there is any conflict, this specification shall prevail.

It should be noted that herein the terms "comprise", "include" or any other variations thereof are intended to cover non-exclusive inclusion, so that a method or device that includes a series of elements not only includes the explicitly stated elements, but also includes other elements that are not expressly listed or that are inherent to the implementation of the method or device. Without further limitation, an element defined by the statement "comprises a..." does not exclude the presence of other related elements in the method or device including the element.

Therefore, a first aspect of the present application provides a compound represented by general formula I:

[M(A)₂]·nB (I)

wherein, M is a divalent metal cation selected from the group consisting of Cu, Fe and Zn;
A is selected from the group consisting of glutathione and a glutathione derivative, wherein the glutathione derivative is selected from the group consisting of glutathione in which at least one of amino group or carboxyl group is substituted by an amino protecting group and/or a carboxyl protecting group;
B is a monovalent or divalent counter anion; and
n is 1 or 2.

According to a specific embodiment, M is Zn²⁺.

The glutathione (GSH) described herein is in L-configuration (L-GSH), that is: N-(N-L-γ-glutamyl-L-cysteinyl)-glycine, having a structural formula as follows:

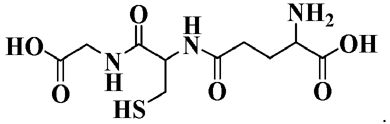

According to a specific embodiment, the amino protecting group is selected from the group consisting of C₁₋₂₀ hydrocarbyloxycarbonyl, C₁₋₆ alkanoyl, C₁₋₆ alkylbenzenesulfonyl, C₁₋₁₀ hydrocarbyl amide and C₁₋₂₀ hydrocarbyl, which are optionally substituted with a substituent selected from the group consisting of halogen, methoxy, ethoxy, methyl, ethyl and a mixture thereof.

The hydrocarbyl can be alkyl, aralkyl, alkenyl, etc.

According to a more specific embodiment, the amino protecting group may be, for example, selected from the group consisting of tert-butyloxycarbonyl (t-Boc), ethoxycarbonyl, methoxycarbonyl, benzyloxycarbonyl (CBz), 2-biphenyl-2-propoxycarbonyl (BPoc), triphenylmethoxycarbonyl (Trt), 9-fluorenylmethoxycarbonyl (Fmoc), allyloxycarbonyl (Al-loc), formyl, acetyl, trifluoroacetyl (Tfa), 2,2-dimethylpropionyl, p-toluenesulfonyl (Tosyl), phthalimido (Pht), methyl, ethyl, propyl, tert-butyl, isopentyl, benzyl, triphenylmethyl (Trityl) and p-methoxybenzyl (Pmb).

Preferably, the amino protecting group can be selected from the group consisting of tert-butyloxycarbonyl (t-Boc) and benzyloxycarbonyl (CBz).

According to a specific embodiment, the carboxyl protecting group is selected from C₁₋₂₀ hydrocarbyloxys.

The hydrocarbyl can be alkyl, aralkyl, alkenyl, etc.

According to a more specific embodiment, the carboxyl protecting group may be, for example, selected from the group consisting of methoxy, ethoxy, propoxy, tert-butoxy, benzyloxy, and 9-fluorenmethoxy.

Preferably, the carboxyl protecting group may be selected from the group consisting of methoxy, ethoxy, and tert-butoxy.

The counter anion may be, for example, a sulfate ion or a halide ion. Examples of a halide ion include a chloride ion and a bromide ion. The counter anion can also be other anions, as long as it is beneficial to obtain the compound and has no adverse effects on its application.

Exemplarily, the compound can be selected from the group consisting of [Zn²⁺(L-GSH)₂]·SO₄²-, [Zn²⁺(L-GSH)₂]·2Cl⁻, [Cu²⁺(L-GSH)₂]·SO₄²⁻, [Cu²⁺(L-GSH)₂]·2Cl⁻, [Fe²⁺(L-GSH)₂]·SO₄²⁻ and [Fe²⁺(L-GSH)₂]·2Cl⁻.

The compound is dissolved in an aqueous solution to form a chelate represented by formula (II): [M(A)₂] (II), where M and A are as defined above.

In formula (II), 2 to 6 coordination bonds are formed between the divalent metal cation M and two molecules of glutathione or glutathione derivative A as a chelating agent. Preferably, 2, 4 or 6 coordination bonds are formed. More preferably, 2 or 4 coordination bonds are formed. The coordination bond is formed between the metal ion M and thiol, amino, imine and/or carboxyl.

The following takes [Zn²⁺(L-GSH)₂], the chelate of zinc ion and glutathione (L-GSH), as an example to illustrate the coordination structure of the compound in an aqueous solution.

Two coordination structure:

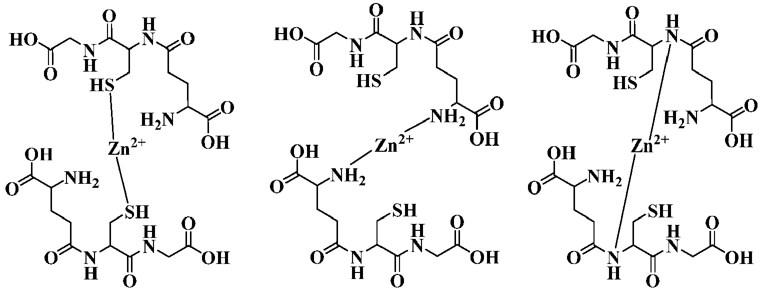

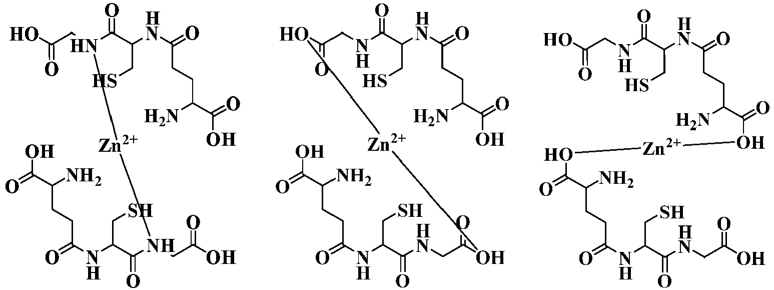

Three coordination structure:

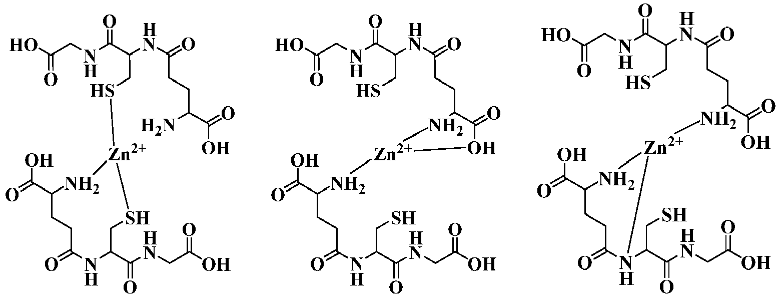

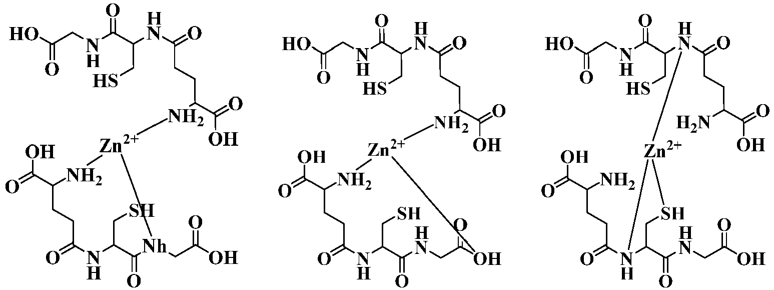

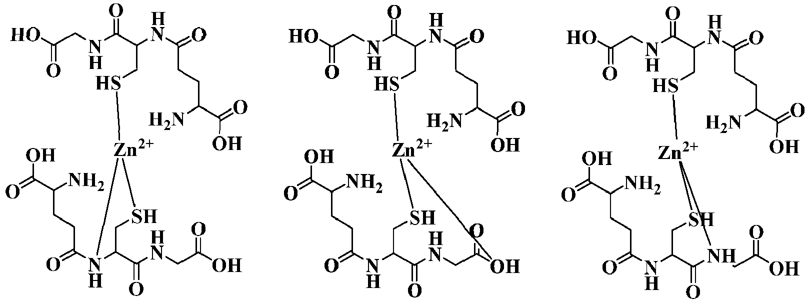

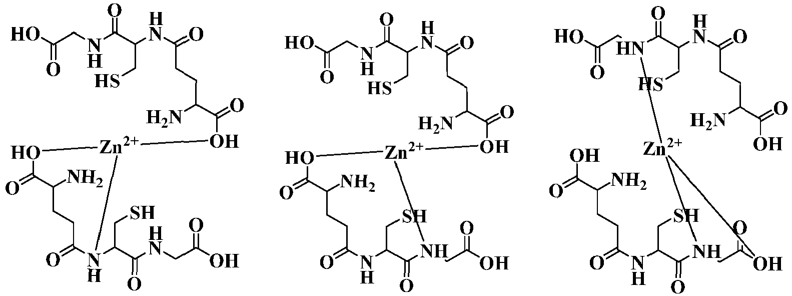

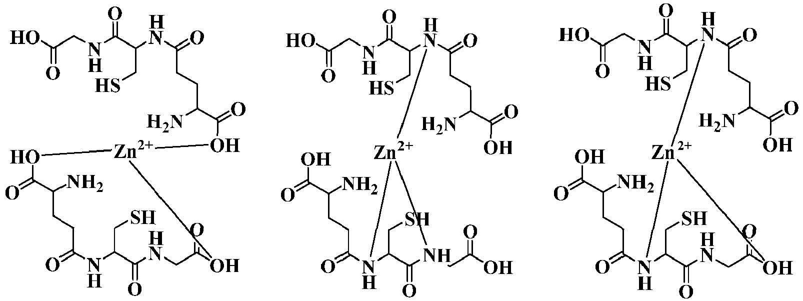

Four coordination structure:

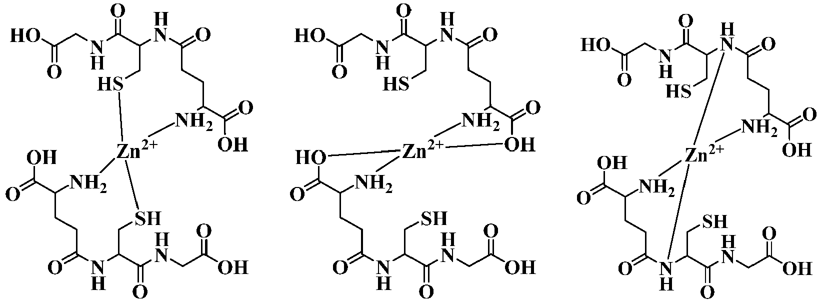

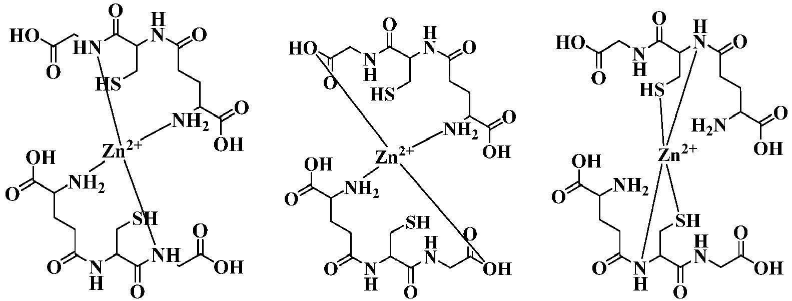

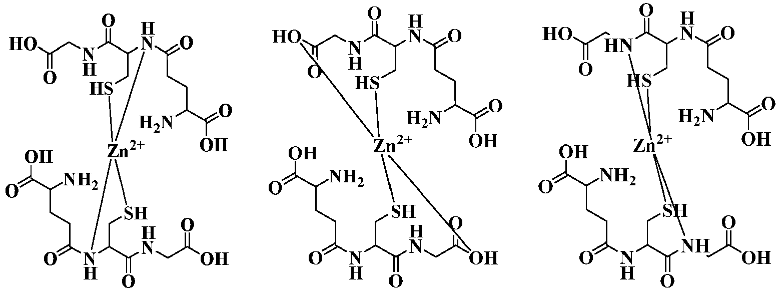

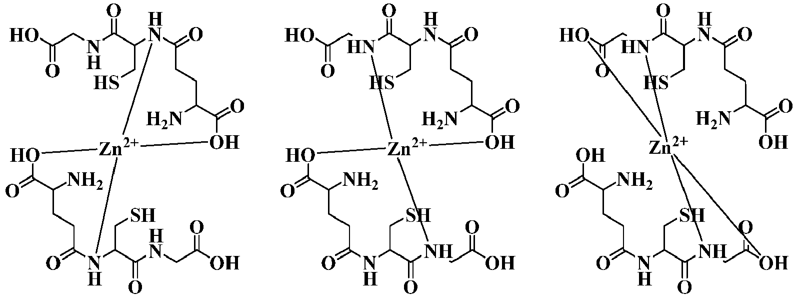

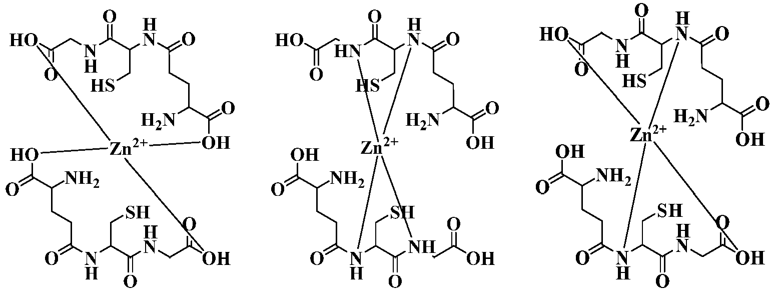

Five coordination structure:

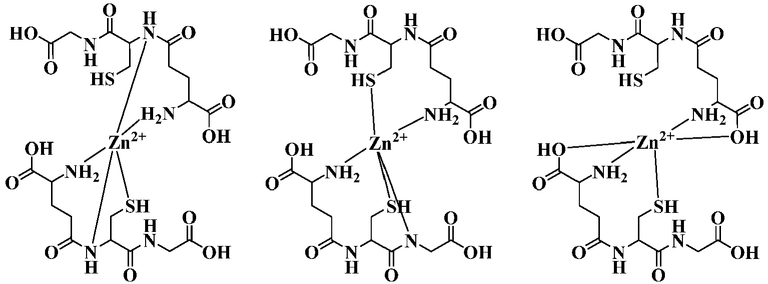

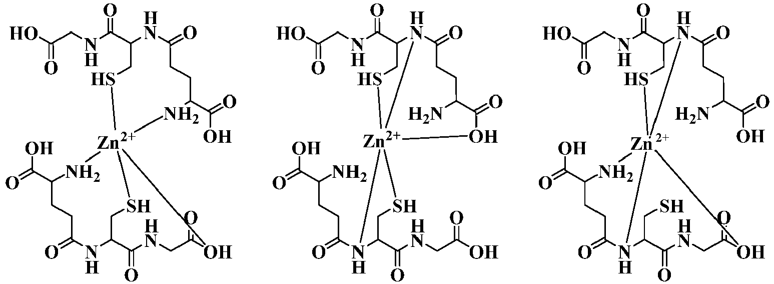

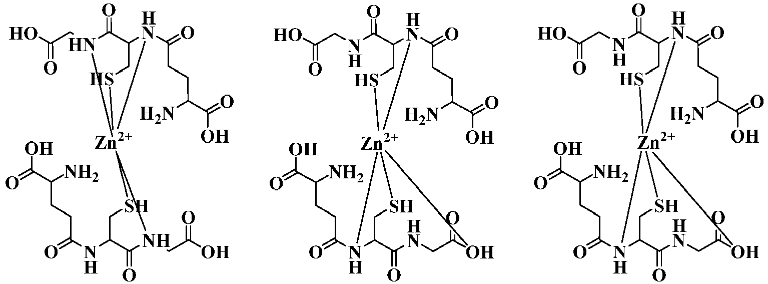

Six coordination structures:

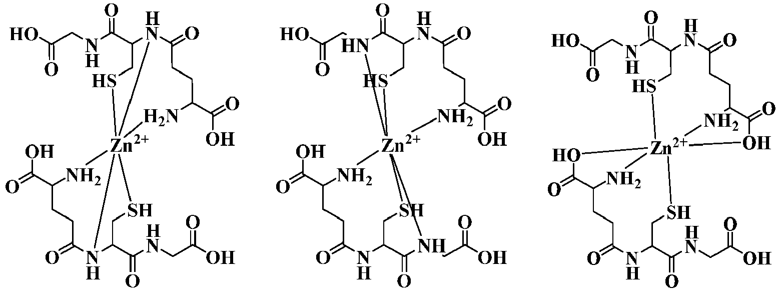

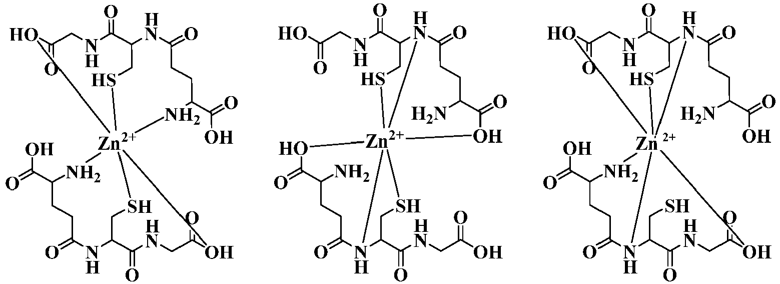

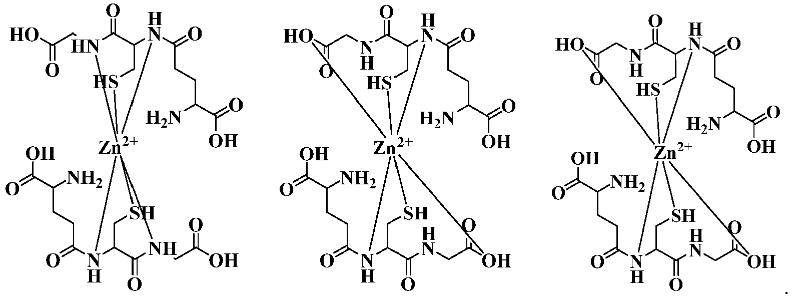

It should be understood that glutathione chelates of copper ions and ferrous ions can have the same structures as described above, that is, Zn²⁺ in the structure [Zn²⁺(L-GSH)₂] shown above can be replaced by Cu²⁺ or Fe²⁺. Moreover, in the structure [Zn²⁺(L-GSH)₂] shown above, one or more (if any) of carboxyl and amino in glutathione that do not form a coordination bond can be substituted by the amino or carboxyl protecting groups defined above.

A second aspect of the present application provides a method for preparing the compound represented by formula (I), comprising mixing A as a chelating agent and the inorganic salt MBₙ of M as a divalent metal cation in an aqueous solvent at a molar ratio of A to MBₙ of 2: (1-1.2) for reaction. The reaction is completed when the reaction solution becomes clear.

Wherein, A, M, B and n are as defined above.

According to a specific embodiment, the inorganic salt MBₙ of the divalent metal cation M may be a hydrochloride salt or sulfate salt of the metal ion M.

According to an embodiment, the aqueous solvent is a mixed solvent of water and alcohol. More particularly, the alcohol is methanol or ethanol. The mixing ratio of water and alcohol is not particularly specified and can be determined based on the solubility of the inorganic salt MBₙ of glutathione or a derivative thereof A and a divalent metal cation. Usually the alcohol can be present in the mixed solvent in a proportion of 10 to 90 vol%. Preferably, the alcohol can be present in the mixed solvent in a proportion of 20 to 80 vol%, 30 to 70 vol%, or even 40 to 60 vol%.

The amount of the aqueous solvent is not particularly specified, as long as the total concentration of the reactants is within an appropriate range.

According to an embodiment, the reaction is carried out under mild conditions. For example, it is carried out at a temperature of 10°C to 60°C. The reaction process can be accelerated when the reaction is carried out at an elevated temperature, such as 40°C to 60°C, but it is still preferably carried out at room temperature.

The duration of the reaction is not particularly limited. The observation that the solution becomes clear indicates that the chelate compound defined above has been formed. For example, stirring can be continued at room temperature for 5 to 20 hours, such as 8 to 12 hours.

Next, the reaction solution is distilled under reduced pressure to obtain a concentrated solution. The concentrated solution is further purified to obtain the compound.

According to a specific embodiment, the purification may comprise dissolving the obtained concentrated solution with a good solvent (such as water), then adding a poor solvent (such as alcohol) to crystallize and precipitate the compound, and then filtering, washing and drying the precipitate.

Other suitable purification methods can also be used for the preparation of the compound, which are not particularly limited by the present application.

A third aspect of the present application provides a pharmaceutical composition comprising a therapeutically effective amount of the above compound.

Wherein, the "therapeutically effective amount" mentioned herein refers to the amount of the compound that has a preventive, alleviating or therapeutic effect on alcoholic liver injury.

The pharmaceutical composition of the present application may further comprise a pharmaceutically acceptable additive, such as an excipient, a solvent and a carrier.

The pharmaceutical composition can be administered to a subject in need thereof by means such as oral administration and parenteral administration. Preferably, it is administered orally.

Another aspect of the present application further provides the above compound or the above pharmaceutical composition for preventing, alleviating or treating alcoholic liver injury.

In addition, the present application further provides a method for preventing, alleviating or treating a subject suffering from alcoholic liver injury, comprising administering to the subject a therapeutically effective amount of the above compound or the above pharmaceutical composition.

A fourth aspect of the present application provides use of the above compound or a pharmaceutically acceptable salt thereof, or the above pharmaceutical composition in the manufacture of a medicament for preventing, alleviating ortreating alcoholic liver injury.

The alcoholic liver injury may be selected from the group consisting of mild alcoholic liver disease, alcoholic fatty liver, alcoholic hepatitis, alcoholic hepatic fibrosis and alcoholic cirrhosis.

Alcoholic liver injury is pathologically different from non-alcoholic liver injury. It is usually caused by a certain number of years of drinking and/or a certain amount of drinking. It is also affected by multiple factors such as gender, race, individual conditions and genetics. Influenced by China's traditional drinking culture and with the improvement of material living standards, the proportion of drinkers in the general population has increased significantly, and the number of people suffering from alcoholic liver injury has been on an upward trend year by year in China.

Alcoholic liver injury can be diagnosed according to clinical classification as the following: mild alcoholic liver disease (MA1), alcoholic fatty liver (AFL), alcoholic hepatitis (AH), alcoholic hepatic fibrosis (AHF) and alcoholic cirrhosis (AC). Among them, mild alcoholic liver disease (MA1) and alcoholic fatty liver (AFL) are mild forms of alcoholic liver disease. Liver fibrosis or hepatic fibrosis is the excessive accumulation of extracellular matrix proteins (including collagen) and the subsequent scarring process. Alcoholic fatty liver is likely to progress to alcoholic hepatic fibrosis. Advanced hepatic fibrosis can progress to cirrhosis overtime. Cirrhosis is the final stage of chronic liver disease and generally has a long-term poor prognosis and is irreversible. Once it develops to the stage of cirrhosis, the damage to the liver is irreversible.

Unfortunately, there are few treatment options available for hepatic fibrosis, and common treatments only target the cause of cirrhosis and/or relieve the symptoms of cirrhosis. For example, tipronin (chemical name: mercaptopropionylglycine) is a metabolismimproving detoxification agent. It is mainly used to improve liver function in acute/chronic liver diseases. It can comprehensively and significantly improve liver function indicators and related symptoms of viral hepatitis and alcoholic liver injury, but has relatively great toxic and side effects. CN105693577A discloses tipronin zinc, and claims that tipronin and zinc have synergistic effects. However, its examples showed that at the same dose, tipronin zinc had basically the same medicinal effect as tipronin, indicating that tipronin zinc did not achieve the synergistic effects it claimed.

Therefore, the prevention, alleviation, treatment, and even cure of early and midstage liver injury are particularly critical and important.

The following detailed examples confirm that the compounds of the present application can effectively reduce liver fat deposition in mice with alcohol-induced liver injury, and can effectively prevent, alleviate and/or treat alcohol-induced liver injury.

Another aspect of the present application provides a food comprising at least one of the compounds represented by formula (I).

The food may be in solid or liquid form. In particular, the food may be a wine. According to a specific embodiment, the alcohol content of the wine is less than or equal to 53 degrees. More specifically, the alcohol content of the wine is 4 to 53 degrees, such as 39 to 53 degrees. The type of the wine is not particularly limited and it can be any kind of wine. White wine is particularly preferred.

Among the compounds of the present application, preferably the food comprises the compound in which M is zinc ion.

According to another specific embodiment, the food comprises a compound selected from the group consisting of [Zn²⁺(L-GSH)₂]·SO₄²⁻, [Zn²⁺(L-GSH)₂]·2Cl⁻, [Cu²⁺(L-GSH)₂]·SO₄²⁻, [Cu²⁺(L-GSH)₂]·2Cl⁻, [Fe²⁺(L-GSH)₂]·SO₄²⁻, [Fe²⁺(L-GSH)₂]·2Cl⁻, and a mixture thereof, particularly preferably comprising [Zn²⁺(L-GSH)₂]SO₄²⁻ or [Zn²⁺(L-GSH)₂]·2Cl⁻

According to a specific embodiment, the wine comprises 06~0.9 g/L, preferably 0.6~0.8 g/L, more preferably 0.7~0.8 g/L, particularly preferably 0.75~0.80 g/L of the compound represented by formula (I).

Particularly, the wine (for example, white wine with an alcohol content of 53 degrees) may comprise 0.784 g/L of the compound represented by formula (I) (especially [Zn²⁺(L-GSH)₂]SO₄²⁻ or [Zn²⁺(L-GSH)₂]·2Cl⁻).

For example, the wine comprising the compound represented by formula (I) can be prepared by adding an appropriate amount of the compound to a wine with an alcohol content of less than or equal to 53 degrees, and leaving the mixture for 7-14 days to allow the compound to be fully mixed with the wine.

Another aspect of the present application provides use of the above compound represented by formula (I) or the above food for preventing and/or alleviating alcoholic liver injury.

The advantages of various aspects of the present application are described in detail below in conjunction with specific examples.

### Example 1 Preparation of L-glutathione-zinc chelate [Zn²⁺(L-GSH)₂]

10 g (2 eq) of L-glutathione (L-GSH) was dissolved in 100 mL of 50% ethanol aqueous solution. When it was completely dissolved, the mixture was slowly added with 5.6 g (1.2 eq) of ZnSO₄·7H₂O, and stirred at room temperature overnight, until the reaction solution became clear. A small amount of the reaction solution was taken several times from the beginning to the end of the reaction, and the changes in the reactants and products were monitored by HPLC (Agilent 1260).

HPLC conditions: (acetonitrile-water elution, acetonitrile content changes (0-5 min, 5%-100%; 5-10 min, 100%); chromatographic column: Thermo Fisher, C18, 100×46 mm, 5 µm; injection volume: 20 µL)

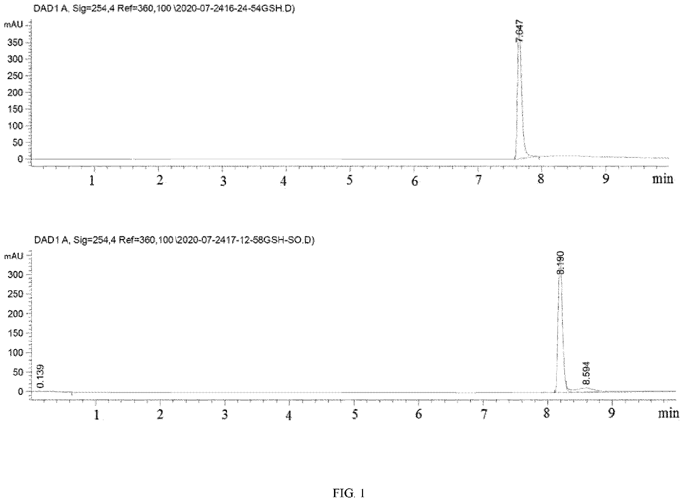

The results are shown in FIG. 1. As can be seen from FIG. 1, only a sharp single peak of L-GSH (t=7.647) is visible at the beginning of the reaction. As time goes by, it can be seen that the single peak broadens, and gradually another peak appears at a longer retention time (t=8.190), indicating that the product is gradually generated. When the peak of L-GSH disappears and only the peak at t=8.190 remains, the reaction is completed.

After the reaction was completed, the reaction solution was concentrated under reduced pressure to obtain a colorless oil. An appropriate amount of water was added to the concentrate to completely dissolve it, and then a large amount of absolute ethanol was added to precipitate the product, which was filtered with suction. The precipitate was washed with absolute ethanol, and dried under vacuum to obtain product (a) in the form of a white solid powder, with a yield of 70% . The purity of product (a) was determined to be 80.0% using liquid chromatography-mass spectrometry.

According to the same method as above, zinc chloride was used instead of zinc sulfate to obtain product (b) in the form of a white solid powder with a yield of 70%. The purity of product (b) was determined to be 84.9% using liquid chromatography-mass spectrometry.

### Example 2 Identification of the product obtained in Example 1

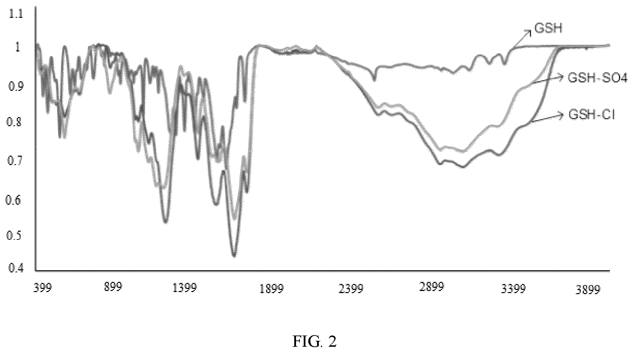

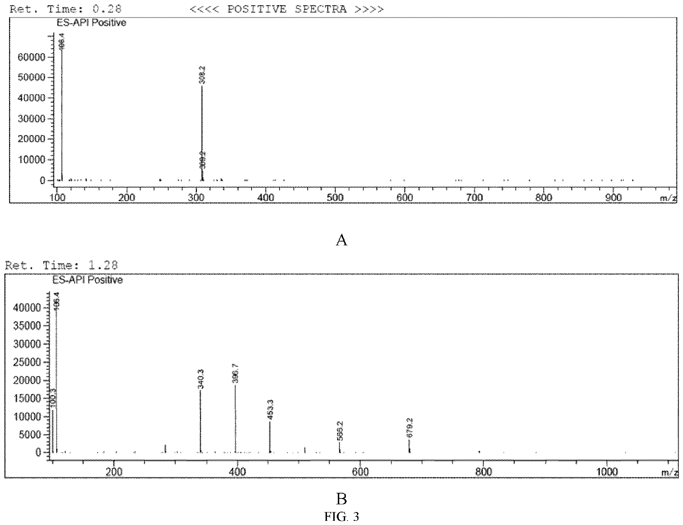

The product (a) and product (b) obtained in Example 1 and pure L-glutathione (purchased from Adamas) were subjected to infrared spectroscopy (Nicolet 6700, American Thermoelectric Company) for detection. The product (a) obtained in Example 1 and pure L-glutathione were detected by mass spectrometry (LCMS 1260-6110, Agilent) to further determine the product structure. The results are shown in FIGs. 2 and 3 respectively.

FIG. 2 shows the infrared spectra of L-GSH, product (a) and product (b) of Example 1. It can be seen that the absorption peaks of L-GSH (curve GSH) and the two products of Example 1 are obviously different. The location and intensity of the infrared absorption peaks of product (a) (curve GSH-SO₄) and product (b) (curve GSH-Cl) are similar. It shows that the use of different zinc salts as reactants leads to L-GSH-Zn chelates with the same structure.

Further, FIG. 3 shows the mass spectra of L-GSH (A in FIG. 3) and product (a) of Example 1 (B in FIG. 3). The mass spectrum of L-GSH shows that m/z is 308.2 (theoretical value is 307.33). B shows the mass spectrum of the product of Example 1, which shows that the maximum m/z is 679.2 (theoretical value of molecular weight of [Zn²⁺(GSH)₂] is 680.05). It indicates that [Zn²⁺(GSH)₂] chelate is prepared by the method of Example 1, and based on the reactants, it can be determined that product (a) is sulfate salt Zn(GSH)₂•SO₄.

### Example 3 Establishment of mouse alcoholic liver injury model

This experiment used the Lieber-DeCarli alcoholic fatty liver modeling method. Lieber-DeCarli feed was purchased from Nantong Trophic Animal Feed High-Tech Co., Ltd. The specific method is as follows: C57 mice (purchased from TROPHIC Animal Feed High-Tech Co. Ltd, China, the same below) were fed with normal solid maintenance feed, adapted to the feeding environment for at least one week, then were fed with maltodextrin diet for a week of transition, and then fed with Lieber-DeCarli feed. The alcohol concentration of Lieber-DeCarli feed was 3% in the first week, and the alcohol concentration was increased by 0.5% every week until reaching an alcohol concentration of 5%. The animals were continued to be fed with feed containing 5% alcohol. C57 mice were fed with Lieber-DeCarli feed for 12 weeks to establish a model of alcoholic liver injury.

According to the material collection and measurement methods in the following Example 4, after the establishment of model was completed, the formation of alcoholic fatty liver, the fat deposition in liver, the changes in transaminase and morphological structure change of tissues, and degree of fibrosis in the livers of animals fed normally and animals fed with Lieber-DeCarli alcohol feed were measured.

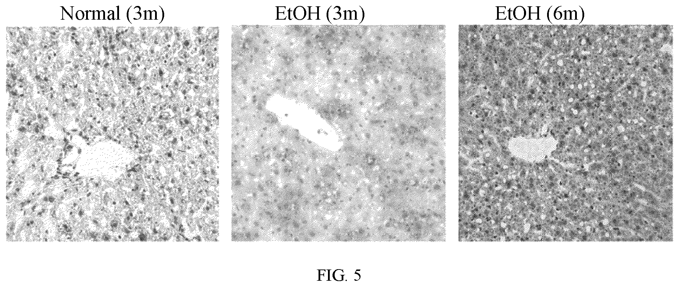

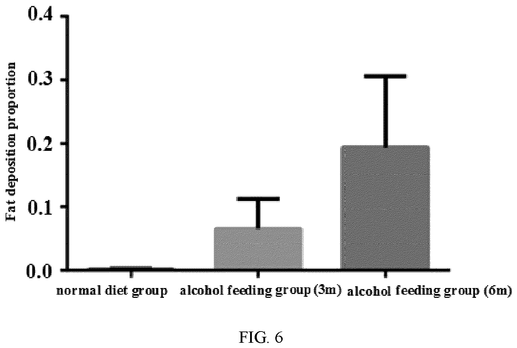

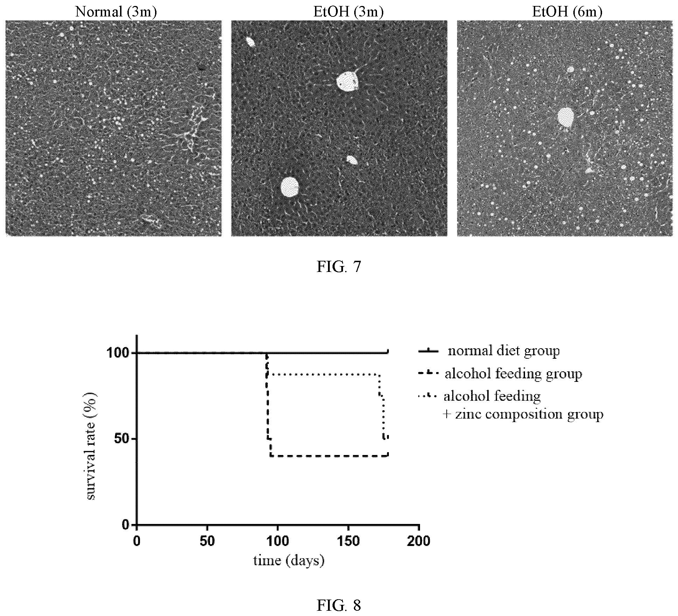

Among them, oil red O-staining of the liver clearly showed that there was obvious fat deposition in the liver of mice fed with Lieber-DeCarli alcohol feed, while no fat deposition was found in the liver of mice fed normally (see FIGs. 5 and 6). Increased vesicular steatosis can also be seen with HE staining (see FIG. 7).

This proves that the model was successfully established.

### Example 4 Effect of product (a) of Example 1 on alcoholic liver injury

### Grouping and processing methods:

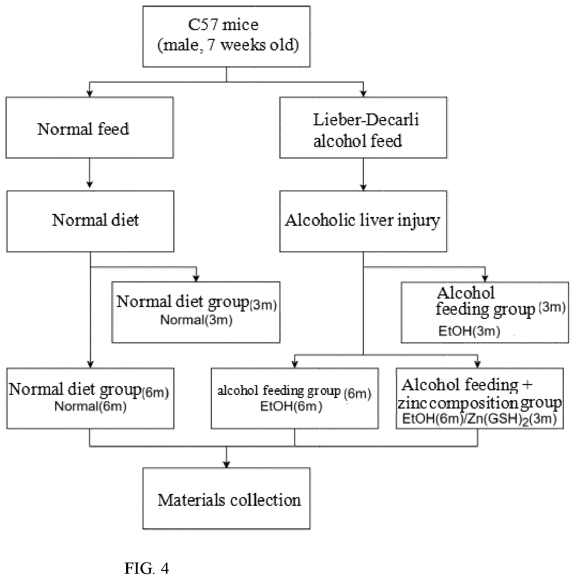

30 C57 mice were subjected to adaptive feeding for 1 week, and then randomly divided into a normal diet group (n=9) and an alcohol diet group (n=21) according to body weight. In the alcohol diet group, a mouse alcoholic liver injury model was established by feeding mice with Lieber-DeCarli feed for 12 weeks according to the method of Example 3. After the modeling was completed, the mice were randomly divided into 3 groups, one of which was the pre-intervention group (EtOH (3m), n=3) (meanwhile, 4 animals were randomly selected from the normal diet group to be control of the pre-intervention group (Normal (3m) group, n=4). The pre-intervention group and the pre-intervention control group were collected immediately after the modeling was completed. The other two groups were the non-intervention group (EtOH (6m), n=10), and the zinc/glutathione intervention group (EtOH(6m)/Zn(GSH)₂(3m), n=8) respectively. The remaining normal diet group was named the normal group (Normal (6m) group, n=5). The detailed process and grouping are shown in FIG. 4.

### Intervention methods:

Zn(GSH)₂•SO₄ was added to the daily Lieber-DeCarli alcohol diet at the amount of 1 mg Zn/10 mL of feed for three months of invention.

### Material collection and measurement methods:

At the end of the experiment in each group, the animals were weighed, blood was collected, and materials were collected.

Blood collection: orbital blood collection was performed.

Liver: The animals were killed by cervical dislocation. The mice were cut along the midline of the abdomen to expose the abdomen and the liver was then peeled off. The intact liver was peeled off and separated into the different lobes. The left lobe was separated into three sections using a blade, and the right lobe was separated into two sections.

Tissue distribution of each lobe of the liver: The left lobe was separated into three sections and the right lobe was separated into two sections. The first sections of the left lobe and right lobe were used to make pathological sections for histological examination. The second sections of the left lobe and right lobe were used for zinc measurement. Other lobes were stored in liquid nitrogen for analysis such as molecular detection.

Biochemical analysis of serum: Aspartate aminotransferase (AST), alanine aminotransferase (ALT), and aspartate aminotransferase/alanine aminotransferase (AST/ALT) in blood samples were analyzed using a colorimetric method.

Oil red O staining of frozen sections: Frozen sections were stained with ORO stain solution and the nuclei were counterstained with hematoxylin. Immediately after being sealed, the slides were observed with a microscope, and the images were collected. The area of the ORO positive area was measured using Image Pro-Plus 6.0 software, and the ratio of the positive area to the total area was calculated.

Sirius red staining: Paraffin sections were stained with Sirius red staining solution. After being sealed, the slides were observed using a 100x microscope, and pictures were taken.

Measurement of zinc concentration: The zinc concentration in liver tissue was determined using atomic absorption spectrometry (atomic absorption spectrometer: ICE3500, Thermo Company).

Data processing and statistical analysis: All experimental data herein were analyzed using GraphPad Prime 6 statistical analysis software. Student t test was used for single factor analysis between two groups, and p < 0.05 was considered to be statistically different.

### Test results:

### 4.1 Survival conditions

According to the aforementioned intervention method, intervention was performed after modeling was completed. The survival rate was calculated. The results are shown in FIG. 8. Under the intervention of zinc complex, the survival rate of mice was significantly increased.

The changes in the average body weight of animals in each group are shown in Table 1 below.

**Table 1**

| Item | Normal(3m) | Normal(6m) | EtOH(6m) | EtOH(6m)/Zn(GSH)₂ |
|---|---|---|---|---|
| Initial body weight (g) | - | - | 22.78±0.52 | 22.93±3.73 |
| Final body weight (g) | 25.24±1.20 | 27.51±1.99 | 24.94±1.73 | 22.53±1.67 |
| Liver weight (g) | 1.30±0.20 | 1.35±0.38 | 1.20±0.13 | 1.26±0.25 |
| Liver weight/final body weight (%) | 5.16±1.02 | 4.88±1.00 | 4.81±0.24 | 5.62±0.80 |

### 4.2 Fat deposition

According to the aforementioned modeling method, calculation was performed based on the oil red O staining results. The results are shown in FIG. 6 below. Compared with the normal diet group (Normal (3m)) as the control, mice fed with alcohol feed for 3 months (EtOH(3m)) and 6 months (non-intervention group EtOH (6m)) showed obvious fat deposition. As duration of feeding increased, fat deposition increased.

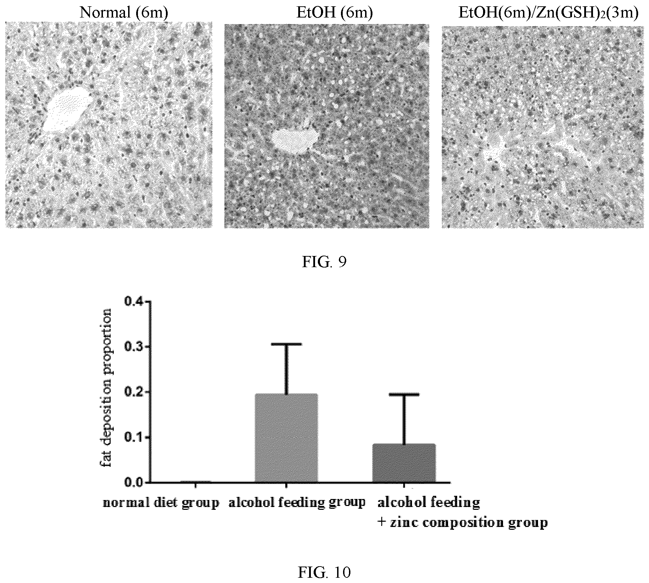

Referring further to FIG. 9, according to the aforementioned intervention method, the fat deposition of the zinc/glutathione group (EtOH(6m)/Zn(GSH)₂(3m)) that was intervened after modeling was significantly improved compared to the non-intervention group.

The percentage of fat deposition in the total liver area was calculated based on the oil red O staining results. The results are shown in FIG. 10 below. As can be seen from FIG. 10, under the action of zinc complex, the area of fat deposition was significantly reduced, and there was a difference compared with the non-intervention group.

### 4.3 Liver injury

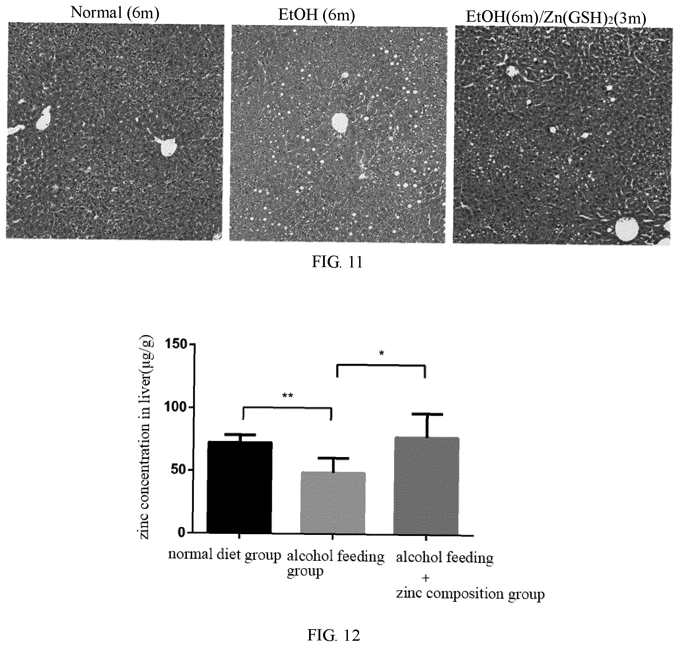

The results of HE staining are shown in FIG. 11: under the action of Zn(GSH)₂•SO₄, vesicular steatosis was significantly reduced compared with the non-intervention group.

### 4.4 Zinc content in liver

The amount of zinc in the livers of mice in each group was measured according to the above method. The results are shown in FIG. 12. As can be seen from FIG. 12, the zinc content in the liver of mice in the non-intervention group was significantly reduced, while the zinc content in the liver of mice in the intervention group was significantly increased.

### Example 5 Preparation of health wine containing glutathione zinc

0.392 g of the glutathione-zinc sulfate prepared in Example 1 was added to 500 ml of 53-degree white wine, and the mixture was left for 10 days for fully mixing to obtain the finished product.

### Example 6 Effect of product (a) of Example 1 on alcoholic liver injury

### Grouping and processing methods:

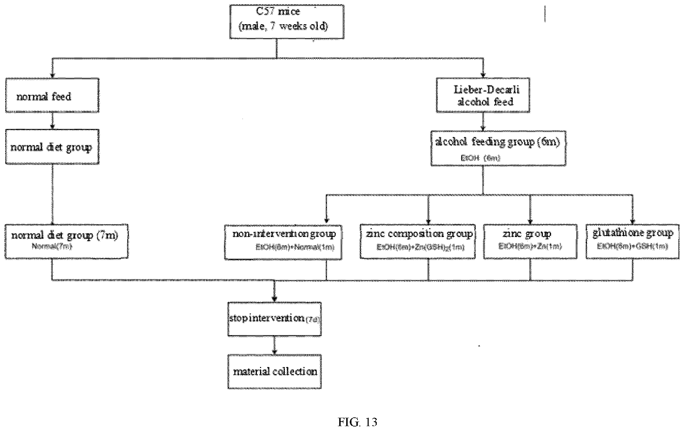

30 C57 mice were subjected to adaptive feeding for 1 week, and then randomly divided into a normal diet group (n=6) and an alcohol diet group (n=24) according to body weight. In the alcohol diet group, a mouse alcoholic liver injury model was established by feeding mice with Lieber-DeCarli feed for 24 weeks according to the method of Example 3. After the modeling was completed, the mice were randomly divided into 4 groups, namely the non-intervention group (EtOH(6m)+Normal(1m), n=6), the zinc glutathione intervention group (EtOH(6m)+Zn(GSH)₂ (1m), n=6), the zinc intervention group (EtOH(6m)+Zn(1m), n=6), and the glutathione intervention group (EtOH(6m)+GSH(1m), n=6). The remaining normal diet group was named the normal group (Normal (7m) group, n=5). The detailed process and grouping are shown in FIG. 13.

### Intervention methods:

Different intervention substances were added to the normal liquid feed for 1 month. The feed of the zinc glutathione intervention group was added with Zn(GSH)₂SO₄ at an amount of 1 mg Zn per 10 mL of feed (GSH was 9.35 mg/10 mL) for one month of intervention. The feed of the zinc intervention group was added with ZnSO₄ at an amount of 1 mg Zn per 10 mL of feed. The feed of the glutathione intervention group was added with glutathione at an amount of 9.35 mg GSH per 10 mL of feed.

### Material collection and measurement methods:

At the end of the experiment in each group, the animals were weighed, blood was collected, and materials were collected.

### Blood collection: orbital blood collection was performed.

Liver: The animals were killed by cervical dislocation. The mice were cut along the midline of the abdomen to expose the abdomen and the liver was then peeled off. The intact liver was peeled off and separated into the different lobes.

Tissue distribution of each lobe of the liver: The left lobe was separated into three sections and the right lobe was separated into two sections. The first sections of the left lobe and right lobe were used to make pathological sections for histological examination. The second sections of the left lobe and right lobe were used for zinc measurement. The remaining third section of the left lobe was stored in liquid nitrogen for analysis such as molecular detection.

Biochemical analysis of serum: Alanine aminotransferase (ALT) in blood samples was analyzed using a colorimetric method.

Oil red O staining of frozen sections: Frozen sections were stained with ORO stain solution and the nuclei were counterstained with hematoxylin. Immediately after being sealed, the slides were observed with a microscope, and the images were collected. The area of the ORO positive area was measured using Image Pro-Plus 6.0 software, and the ratio of the positive area to the total area was calculated.

Data processing and statistical analysis: All experimental data herein were analyzed using GraphPad Prime6 statistical analysis software. Student t test was used for single factor analysis between two groups, and p < 0.05 was considered to be statistically different.

### Test results:

### 4.1 Alanine aminotransferase (ALT)

Determination method: After blood was collected, the collected whole blood was placed at 25°C for 30 minutes. After the blood coagulated, it was centrifuged at 3500 rpm for 15 minutes to separate the serum, and various parameters were measured timely. ALT in the serum was measured using a kit (Nanjing Jiancheng Bioengineering Institute), and the operation was performed according to the product instructions.

### The measurement results are shown in FIG. 14.

In the alcohol diet groups with six months of alcohol treatment, the ALT content in the serum of the mice in the non-intervention group that had been fed with normal feed for one month increased from 12.03 to 44.84, compared with the normal diet group that had been fed with normal feed, which was increased by more than 2.6 times, suggesting that liver injury persist in mice. The ALT level of the zinc glutathione intervention group (8.96) was significantly lower than that of the non-intervention group (44.84), and returned to a normal level. Although the ALT levels of the zinc intervention group (22.03) and the glutathione intervention group (22.86) were both lower than that of the non-intervention group (44.84), the ALT levels did not return to a normal level, which were about 2 times of that of the zinc glutathione intervention group.

### 4.2 Fat deposition

The results of oil red O staining are shown in FIG. 15. Compared with the normal diet group as a control, mice in the non-intervention group showed significant fat deposition.

The percentage of fat deposition in the total liver area was calculated based on the oil red O staining results. The results are shown in FIG. 16 below. The proportion of fat deposition in the zinc glutathione intervention group (0.21) was significantly improved compared with the non-intervention group (0.68). The proportion of fat deposition in the zinc intervention group (0.36) was improved compared with the non-intervention group (0.68), but the effect was not as good as that of the zinc glutathione intervention group. There was no significant difference between the proportion of fat deposition in the glutathione intervention group (0.62) and that of the non-intervention group (0.68).

The above are only preferred embodiments of the present invention, and do not limit the patent scope of the present invention. Under the inventive concept of the present invention, equivalent structural transformations made by using the contents of the description and drawings of the present invention, or direct/indirect applications in other related technical fields are all included in the patent protection scope of the present invention.

## Claims

1. A compound represented by the following formula (I):
[M(A)₂]·nB (I)
wherein, M is a divalent metal cation selected from the group consisting of Cu, Fe and Zn;
A is selected from the group consisting of glutathione and a glutathione derivative, wherein the glutathione derivative is selected from the group consisting of glutathione in which at least one of amino group or carboxyl group is substituted by an amino protecting group and/or a carboxyl protecting group;
B is a monovalent or divalent counter anion; and
n is 1 or 2.

2. The compound according to claim 1, wherein B is a sulfate ion or a halide ion.

3. The compound according to claim 1 or 2, wherein the glutathione and glutathione derivative are formed from an L-type amino acid.

4. The compound according to claim 1, which is selected from the group consisting of [Zn²⁺(L-GSH)₂]·SO₄²⁻, [Zn²⁺(L-GSH)₂]·2Cl⁻, [Cu²+(L-GSH)₂)·SO₄²⁻, [Cu²⁺(L-GSH)₂]·2Cl⁻, [Fe²⁺(L-GSH)₂]·SO₄²⁻ and [Fe²⁺(L-GSH)₂]·2Cl⁻.

5. A method for preparing the compound according to any one of claims 1 to 4, comprising:
mixing A and an inorganic salt MBₙ of M in an aqueous solvent at a molar ratio of A to MBₙ of 2: (1-1.2) for reaction.

6. A pharmaceutical composition comprising a therapeutically effective amount for treating alcoholic liver injury of the compound according to any one of claims 1 to 4.

7. A food comprising the compound according to any one of claims 1 to 4.

8. The food according to claim 7, which is a wine, preferably with an alcohol content of less than or equal to 53 degrees.

9. The food according to claim 8, wherein the wine comprises 0.6 to 0.8 g/L of the compound.

10. Use of the compound according to any one of claims 1 to 4 or the pharmaceutical composition according to claim 6 in the manufacture of a medicament for preventing, alleviating or treating alcoholic liver injury.
